**Europäisches Patentamt**

(19) **European Patent Office**

**Office européen des brevets**

(11) Publication number: **0 325 492 B1**

(12) **EUROPEAN PATENT SPECIFICATION**

(45) Date of publication of patent specification :
27.05.92 Bulletin 92/22

(51) Int. Cl.⁵ : **A61K 9/22**

(21) Application number : **89300582.7**

(22) Date of filing : **23.01.89**

(54) **Osmotic system for delivery of dilute solutions.**

(30) Priority : **22.01.88 US 147182**

(43) Date of publication of application :
**26.07.89 Bulletin 89/30**

(45) Publication of the grant of the patent :
**27.05.92 Bulletin 92/22**

(84) Designated Contracting States :
**AT BE CH DE ES FR GB GR IT LI LU NL SE**

(56) References cited :
**US-A- 4 278 087**

(73) Proprietor : **BEND RESEARCH, INC.**
**64550 Research Road**
**Bend Oregon, 97701 (US)**

(72) Inventor : **Smith, Kelly L.**
**19295 Dayton Road**
**Bend Oregon 97701 (US)**
Inventor : **Robinson, Wendy P.**
**Apartment 40 1420 Lake Boulevard**
**Davis California 95616 (US)**

(74) Representative : **Skerrett, John Norton Haigh et al**
**H.N. & W.S. Skerrett Charles House 148/9**
**Great Charles Street**
**Birmingham B3 3HT (GB)**

**Description**

BACKGROUND OF THE INVENTION

Systems for the delivery of beneficial agents are well know in the art. For example, dispensing systems that deliver their contents by diffusion through a permeable polymer coating or wall are well known, but suffer from severe limitations. A key limitation is that many beneficial agents cannot be delivered from such diffusion-controlled devices at useful rates. In many instances, permeation rates through the permeable polymer coating are inadequate due to the solubility properties of the agents or due to the high molecular weight of the agents.

Also well known are delivery systems that operate by means of an osmotic pumping mechanism to achieve high release rates of water-soluble agents. In a typical delivery system of this type, the agent is contained within a continuous, semipermeable coating having a hole of predetermined size drilled therethrough. See, for example, U.S. Patent Nos. 3,845,770, 3,916,899, 4,008,719, 4,014,334, 4,016,880, 4,034,758 and 4,077,407. In operation, the delivery system is placed in an appropriate aqueous environment, whereupon it imbibes water through the semipermeable coating, thereby dissolving at least in part the contents of the delivery system. This osmotic influx of water causes an increase in the internal pressure of the system, which results in the dissolved contents being continuously pumped out of the delivery system through the hole at a controlled rate over a prolonged period of time.

Such systems have a number of deficiencies. They are, in most cases, complex devices having multiple parts or requiring special fabrication steps. Consequently, the delivery system is relatively expensive to fabricate and is suited only to relatively large devices, rather than to small beads or prills. Prior art devices having a single hole through which their dissolved contents are delivered suffer from the disadvantage of delivering only saturated solutions of the agent, if the delivery rate is to be maintained constant. Such high concentrations localized near the delivery hole can cause tissue irritation or ulceration if the device is present inside the body, or toxic effects to plants or animals if the device is present in or near such organisms. For example, drugs such as potassium chloride, aspirin, and indomethacin can cause gastrointestinal irritation or bleeding in concentrated solutions.

More recent osmotic delivery systems disclose means of alleviating some of the disadvantages of delivering saturated solutions from a single delivery hole. For example, U.S. Patent No. 4,298,003 described a means for dilution of the saturated agent solution prior to its delivery from a device with a bifurcated reservoir containing soluble agent and an insoluble compound. This improvement, while potentially important in reducing irritation or toxicity, is not useful for delivery small quantities of highly water-soluble agents at constant rates, and therefore suffers from lack of general application. In addition, it is not useful for delivery of more than one agent at independently controlled rates. U.S. Patent No. 4,200,098 discloses a device comprising an osmotically pumping core situated within a distribution zone comprising a layer of water-soluble salts or sugars, the core and distribution zone in turn being surrounded by a wall of microporous or hydrogel material. Although this device theoretically provides two levels of rate control by the membranes of both the osmotic pump portion and the outer wall, due to the porosity of the outer wall, increased porosity leads to an increased rate of release, but at the same time causes a greater degree of dilution of agent that is released, effectively negating this element of control.

Still another type of osmotic device is disclosed in U.S. Patent No. 4,278,087, which discloses agent depots dispersed in a polymer matrix that is impermeable to the passage of agent and permeable to the passage of fluid, the agent depots themselves being surrounded individually with a layer of the same polymer. Upon imbibation of water, the agent depots swell and burst, forming apertures in the polymer surrounding the agent depots, thereby permitting release of agent that is diluted by the imbibed water.

In summary, prior art devices generally are capable of delivering a less-than-saturated solution of agent at a non-constant rate or a saturated solution at a constant rate, but not a less-than saturated solution at a constant rate. The last-mentioned possibility can now be achieved with the present invention.

SUMMARY OF THE INVENTION

The present invention comprises both a unique device and a system for the controlled release of a less-than-saturated, or dilute solution of an active ingredient or agent. The device is osmotically-driven, and comprises agent-containing bodies in a reservoir defined by a surrounding wall, the wall generally being substantially impermeable to agent. However, at least part of the wall is a semipermeable membrane which is "semipermeable" in the sense that it is substantially more permeable to water than to agent; in addition, a portion of the reservoir wall may be essentially impermeable to agent and to water. The wall has one or more delivery ports therethrough.

In the present invention, there are essentially-two types of agent-containing bodies;
(1) those with a release mechanism comprising osmotic pumping of a solution of agent (hereinafter referred to as "osmotic bodies"), and (2) those with a release mechanism comprising diffusion of pure agent through a membrane (hereinbefore "diffusional bodies"). It is thus apparent that release of agent from each of the two types of agent-containing bodies is controlled by a rate-controlling membrane. In the case of the osmotic body, the rate-controlling membrane is also "semipermeable" in the sense that it is substantially more permeable to water than to beneficial agent. In the case of the diffusional body, the rate-controlling membrane is permeable to agent.

More particularly, the invention provides a device for the controlled release of a less-than-saturated aqueous solution of at least one beneficial agent comprising a reservoir defined by a surrounding wall that is substantially impermeable to said agent, at least a portion of said surrounding wall comprising a membrane that is substantially more permeable to water than to said beneficial agent, said wall further having at least one agent delivery port therethrough, characterised in that said reservoir contains at least one agent-containing body, said body comprising a core containing a beneficial agent, said core being surrounded by a rate-controlling membrane, and said rate-controlling membrane is selected from type (a), comprising a semipermeable membrane that is substantially more permeable to water than to said beneficial agent with at least one preexisting delivery port therethrough; and type (b), comprising an agent-permeable membrane that is either porous or non-porous.

The agent-containing bodies may be of virtually any shape or size or number so long as they fit within the reservoir of the device, including, but not limited to, particles, granules, beads, tablets and larger masses. Agent may be present in the reservoir apart from the agent-containing bodies as well (hereinafter "free agent" or "free beneficial agent"). The reservoir may also contain soluble or insoluble filler. In the case of osmotic bodies, each body comprises a core containing the agent and surrounded by a semipermeable membrane having one or more delivery ports through the membrane. In the case of diffusional bodies, each body comprises a core containing the agent and surrounded by an agent-permeable porous or non-porous membrane.

In operation, water is imbibed, for example, from the environment of use and diffused through the reservoir's semipermeable membrane, where it dilutes agent released from the agent-containing bodies and at the same time dissolves any free agent present in the reservoir. In the case of osmotic bodies, imbibed water diffuses though the semipermeable membrane of a given osmotic body containing the agent so as to dissolve some of the agent; the resulting saturated solution of agent is then released by osmotic pumping through the port(s) of the body's membrane and into the reservoir where it is diluted by the imbibed water, and then further driven by osmotic pumping due to the difference in osmotic pressure between the reservoir and the environment of use external to the reservoir through the reservoir's port(s) into the environment of use. In the case of diffusional bodies, imbibed water dilutes the agent released from the diffusional bodies, which dilute solution of agent is driven by osmotic pumping through the reservoir's port(s) into the environment of use in the same manner as in the case of osmotic bodies.

Within a short time of the device's initial contact with water, the concentration of agent within the device reaches a steady value, as do the rate of diffusion of water into the reservoir and the rate of agent release from the agent-containing bodies into the reservoir; the steady state of these kinetics causes the overall rate of release of agent to become constant. The time to reach such a steady state can be shortened by including an appropriate quantity of free agent in the reservoir, which dissolves rapidly as water diffuses into the reservoir.

## BRIEF DESCRIPTION OF THE DRAWINGS

FIGS. 1-6 comprise schematic drawings of exemplary devices of the present invention.

FIGS. 7-9 are graphs showing the controlled release of various beneficial agents from devices of the present invention.

## DETAILED DESCRIPTION OF THE INVENTION

According to the present invention, there is provided a means for dispensing, at substantially constant rates and for predetermined times, beneficial agents, either singly or in combinations, at less-than-saturated concentrations. The agents in the reservoir may be in solid or liquid form, or may comprise the osmotic bodies themselves, so long as they are water-soluble to some extent. Such beneficial agents may include drugs, contraceptives, fertility inhibitors, cosmetics, pheromones, nutrients, vitamins, preservatives, antioxidants, flavors, or other beneficial agents for humans, animals, avians, reptiles an fish; fertilizers, insecticides, pesticides, herbicides, fungicides, acaricides, algicides, growth regulators or promotants, or other biologically active agents for agricultural or horticultural applications; fragrances, disinfectants, colors, indicators, biocides, bactericides, bleaches, reactants, catalysts, air purifiers or other beneficial agents for household or industrial applications.

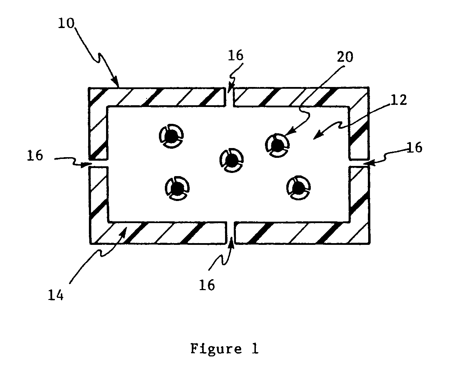

Referring now to the schematic drawings FIGS. 1-6, wherein like numerals designate the same features, and specifically to FIG. 1, one form of the controlled release device 10 of the present invention comprises a reservoir 12 defined by a wall 14, which in this case comprises a semipermeable membrane having one or more ports 16 therethrough. The reservoir has dispersed therein a multiplicity of osmotic bodies 20. In a typical reservoir in tablet form for human use having a volume of 3ml (cc), beads may be present in a number ranging from 1 to 1000.

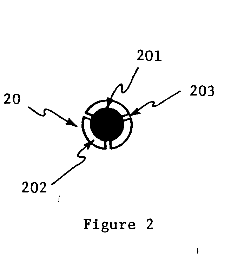

FIG. 2 comprises an enlarged schematic showing the construction of the osmotic bodies. There a body 20 is shown as a core 201 comprising beneficial agent with or without other materials and surrounded by a semipermeable membrane 202 with one or more ports 203 therethrough.

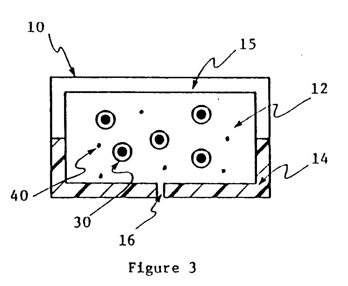

FIG. 3 illustrates another form of controlled release device 10 of the present invention comprising a reservoir 12 defined by a wall comprising partly a semipermeable membrane 14 and partly an impermeable membrane 15, the semipermeable portion 14 of the wall having at least one port 16 therethrough. Within the reservoir 12 are diffusional bodies 30, together with droplets or particles of free agent 40.

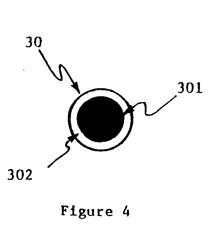

FIG. 4 is an enlarged schematic showing the construction of the diffusional bodies. There a body 30 is shown as a core comprising beneficial agent with or without other materials surrounded by an agent-permeable membrane 302 that may be porous or nonporous.

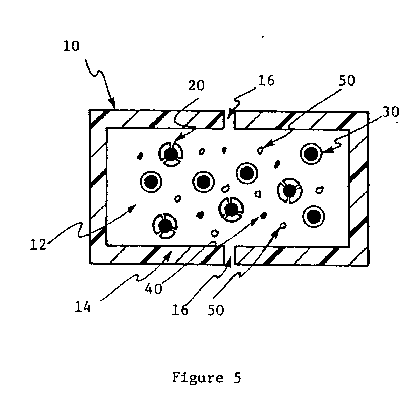

FIG. 5 illustrates another exemplary controlled-release device of the present invention wherein the reservoir 12 is surrounded by a semipermeable wall 14 with ports 16 therethrough, the reservoir containing both osmotic bodies 20 and diffusional bodies 30, together with free agent 40 and filler particles 50.

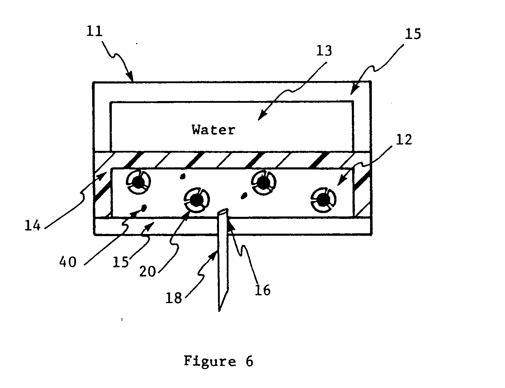

FIG. 6 illustrates still another exemplary controlled-release device 11 of the present invention comprising a reservoir 12 surrounded by a wall comprising partly a semipermeable membrane 14 and partly an impermeable membrane 15, the impermeable portion 15 of the wall having one or more ports 16 therethrough with a needle or cannula 18 extending therethrough for subcutaneous or intramuscular delivery of beneficial agent to the blood stream or to other specific sites on or in a mammal's body, such as at the base of the brain or the locus of a tumor. The reservoir 12 is shown as containing osmotic bodies 20 and free agent droplets or particles 40, and is immediately proximate to a second reservoir 13 surrounded by an impermeable membrane 15 and containing water for imbibition through the semipermeable membrane 14. The device 11 may be equipped with a strap or adhesive (not shown) for attachment to, for example, a patient's arm, and further may be equipped with an activating mechanism such that semipermeable membrane 14 is normally kept segregated from the water in reservoir 13, but which, upon activation of the activating mechanism, is exposed to the water to start the osmotic pumping action.

Exemplary materials for the semipermeable membranes 14 and 202 around both the osmotic bodies 20 and the reservoir 12 include cellulose esters such as mono-, di- and triacylates including mixed esters, regenerated cellulose, cellulose ethers such as ethyl cellulose, polyesters, nylons, polyamides, polycarbonates, polyurethanes, poly(dialkylsiloxanes), poly(methacrylic acid) esters, poly(acrylic acid) esters, poly(phenylene oxides), poly(vinyl alcohols), ethylene/ vinyl alcohol copolymers, aromatic nitrogen-containing polymers, polymeric epoxides, copolymers and blends of any of the foregoing, as well as other film-forming materials suitable for use in reverse osmosis or dialysis applications. Some examples of such suitable film-forming materials include cellulose acetate, cellulose diacetate, cellulose triacetate, cellulose propionate, cellulose acetate butyrate, cellulose acetate propionate, cellulose tripropionate, ethyl cellulose, nylon 6 and the like. Further examples of such semipermeable film-forming polymers are set forth in U.S. Patent No. 4,200,098 at columns 8-10, which disclosure is incorporated herein by reference. Still other film-forming polymers that may be included in the semipermeable membrane of the present invention include agar-agar acetate, acylated alginates, amylose triacetate, beta glucan acetate, beta glucan triacetate, acetyl alginate, triacetate of locust bean gum, alkanoyl carrageenan, acylated tragacanth and esterified gum karaya. The film coating, in addition to being semipermeable, must not adversely affect the beneficial agent or the user of the device. The port(s) through the semipermeable membranes around both the osmotic beads and the reservoir range in diameter from 0.001mm (1 micron) to 1mm; the thickness of the membranes may range from 0.005mm (5 microns) to 1mm.

Plasticizers may also be included in the membrane of the present invention. Such plasticizers include glycols such as glycerol, propylene glycol and polyethylene glycols (MW 200-10,000) and esters such as the phthalates, phosphates, citrates, adipates, tartarates, sebacates, succinates, glycolates, glycerolates, benzoates and myristates, sulfonamides and the like. Specific examples of esters include dimethyl phthalate, dipropyl phthalate, di-(2-ethylhexyl) phthalate, tributyl phosphate, triacetyl phosphate, and tributyl citrate. The plasticizer must be compatible with the other materials of the film coating. It should also have a high degree of permanence, i.e., it should remain in the polymeric film and not migrate to the surface to an appreciable extent. It should also have no adverse effect on the beneficial agent or any animal receiving the device.

The agent-permeable membranes 302 surrounding the diffusional bodies 30 are selected according to their respective permeabilities to the desired agent(s), and may include the same materials that are suitable for the semipermeable membranes 14 and 202, as well as polyethylene, polypropylene, ethylene/vinyl acetate copolymers, silicone rubbers, neoprene rubber, chlorinated polyethylene, polyvinylchloride, vinylchloride copolymers with vinyl acetate, vinylidene chloride, ethylene, and propylene, polyethylene terephthalate, butyl rubber, epichlorohydrin rubbers, ethylene/vinyl alcohol copolymers, polystyrene/ acrylonitrile copolymers, polyamides, polyurethanes, polyesters, and the like. Such agent-permeable membranes may range in thickness from 0.001mm (1 micron) to 2mm, and may be either non-porous or 5% to 95% microporous; in the latter case, the pores may range in diameter from 0.0001 to 0.5mm (0.1 to 500 microns).

Both the diffusional bodies and the osmotic bodies of the present invention may be in essentially four different forms. They may be pure beneficial agent. They may contain soluble filler materials of the types noted below to, for example, increase the osmotic pressure within the reservoir. They may contain insoluble filler materials of the types noted below, which have no effect on osmotic pressure. Or they may comprise soluble or insoluble prills coated with beneficial agent.

In addition to osmotic or diffusional bodies and agent, the reservoir may contain soluble or insoluble filler materials; examples of soluble materials include conventional fillers known in the art of tableting, e.g., salts, carbohydrates such as sugars or starches, alcohols such as mannitol or sorbitol, urea, magnesium succinate, tartaric acid, etc. Examples of insoluble filler materials include conventional tableting fillers such as starches, microcrystalline cellulose and talc. Fillers may be present in amounts ranging from 10 wt% to 90 wt% of the total contents of the reservoir.

There are several levels of control inherent in the device. First, the rate of release of agent from the device is the product of the volumetric flow rate of the solution and the concentration of agent in that solution. The volumetric flow rate of the solution from the device is controlled by the permeability and thickness of the membrane to water and by the osmotic pressure of the solution within the reservoir. The osmotic pressure of the solution within the reservoir is controlled by the concentration of agent and other soluble fillers that may be present in the reservoir. The concentration of agent within the reservoir at steady state in the case of osmotic bodies is controlled by the water permeability and thickness of the membrane surrounding the osmotic bodies, by the solubility of the agent and filler materials, which determines the osmotic pressure within the osmotic bodies, and by the rate of diffusion of water into the reservoir. In the case of diffusional bodies, the concentration of agent within the reservoir at steady state is controlled by the agent permeability and thickness of the permeable membrane surrounding the diffusional bodies. Thus, by varying (1) the types of agent-containing bodies, (2) the permeabilities and thicknesses of the membranes surrounding the agent-containing bodies and the device itself, (3) the number and content of the agent-containing bodies within the reservoir, and (4) the composition of the agent and/or filler within the reservoir, the overall rate and concentration of solution released can be controlled over a wide range.

Several advantages of this device over the prior art are realized. First, the agent concentration in the delivered solution can be varied from nearly zero to saturation, while maintaining a constant rate of release. In prior art devices, only saturated solutions can be delivered at a constant rate of release.

A second advantage is that very small quantities of highly water-soluble agents can be delivered at a constant rate of release over an extended period of time. This could be accomplished by, for example, placing into the reservoir only one or a very few osmotic beads that release the agent very slowly at a constant rate. In prior art devices, such small quantities of agent within the reservoir quickly dissolve, and the concentration decreases with time, resulting in a decreasing release rate with time.

A third advantage is that virtually any number of different agents can be delivered together from the device at independently controlled rates of release and concentrations. This may be accomplished by preparing agent-containing bodies with different membranes and/or different osmotic pressures for each agent and then placing the appropriate number of bodies of each agent into the reservoir. For example, a hydrophilic agent may comprise the core of an osmotic bead, while a hydrophobic agent may comprise the core of a diffusional body, there being an appropriate number of each type of body placed in the reservoir of a device to achieve the desired effect. Prior art devices can only deliver multiple agents at a single ratio equal to their ratio of solubilities. For example, if the desired agents to be delivered are chlorpheniramine maleate and vitamin $B_{12}$, the respective solubilities in water of which are 240 g/L and 12 g/L, the ratio of delivery is fixed at 240/12, or 20:1. In other words the rate of delivery of chlorpheniramine maleate would always be 20 times faster than that of vitamin $B_{12}$. By way of contrast, with a controlled-release device of the present invention, the rate of simultaneous delivery of the same two drugs can be varied significantly by adjusting the amount and type of bodies containing the respective beneficial agents, as demonstrated in Example 5 below.

A fourth advantage is that multiple agents that are incompatible with each other can be delivered from this device, and are isolated from each other during storage by the membrane surrounding each bead.

The device of the present invention is conveniently prepared by conventional techniques. The simplest method comprises conventional overcoating a granule or particle of agent with a semipermeable or agent-permeable membrane. Osmotic or diffusional bodies may also be prepared by coating pharmaceutical prills with a mixture of active agent and polymer by, for example, solvent evaporation coating in a commercial fluid-bed coater to the desired thickness, then overcoating by, for example, solvent evaporation coating, the entire external surface of the agent-coated bead with a semipermeable membrane containing delivery port(s). Other conventional methods of coating with a semipermeable membrane include molding, spraying and dipping.

The delivery port(s) may be formed by including material within the membrane which, upon exposure to water, leaches out or dissolves away, leaving the desired degree of porosity. The pore-forming material is preferably particulate in nature, with a maximum particle size preferably not exceeding about 0.5mm (500 microns) in its longest dimension and an average particle size from about 0.001 mm (1 micron) to about 0.3mm (300 microns), more preferably having an average particle size from about 0.005mm (5 microns) to about 0.1mm (100 microns). It must be soluble in water or aqueous media and insoluble in the organic solvent in which the polymeric film-forming material is dissolved during the film-coating process. Suitable pore-forming materials include water-soluble sugars, e.g., lactose, sucrose, sorbitol and mannitol; water-soluble salts, e.q., alkali metal-, alkaline earth metal-, and transition metal halides, carbonates, phosphates, sulfates, benzoates, acetates, citrates, nitrates, and the like; diols and polyols such as polyhydric alcohols, polyalkylene glycols, polyglycols and poly($\alpha,\omega$)alkylenediols. When the device of this invention is intended for pharmaceutical use, the pore-forming material must be pharmaceutically acceptable. A portion of the beneficial agent may be used as the pore-forming material, and in certain formulations this may be preferred. The delivery port(s) may also be formed by mechanical or laser drilling, depending upon size, economic factors, etc.

In the case of diffusional bodies, the core may also comprise pure agent, agent with other material such as soluble or insoluble filler, or an agent-coated prill, which is then overcoated with agent-permeable membrane by the same conventional techniques mentioned above.

The agent-containing bodies may then beplaced in the reservoir portion of the device by, for example, mixing the bodies with tableting excipients and compressing the mixture into tablets, followed by coating the external surface of the tablet with the semipermeable membrane containing the delivery ports or water-leachable delivery ports. The delivery ports in the walls of the device, whether semipermeable or impermeable, may also be prepared by the same techniques mentioned above as to osmotic bodies.

Referring to the type of device illustrated in FIG. 6, the agent-containing bodies together with free agent or filler or both, if desired, may be loaded onto the impermeable portion of the device's wall. The semipermeable portion of the wall may then be heat-sealed over the impermeable portion, thereby defining the reservoir. Delivery ports through the semipermeable portion of the wall may be made by the same techniques noted above.

The impermeable backing 15 may be made of metal such as thin-gauge aluminium or steel or other material such as plastics like nylon, polysulfone, or polycarbonate.

Example 1

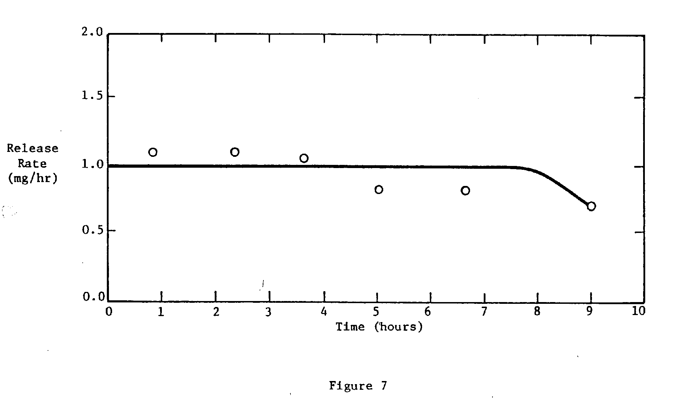

Osmotic bodies were prepared by coating in a fluid-bed coater 10 g of 18-20 mesh (in terms of the Standard mesh system) sucrose/starch seed prills (nonpareils) with a mixture of 2 g pyridostigmine bromide (a cholinegic drug) and 0.8 g cellulose acetate manufactured and sold by Eastman Kodak Co. under the designation CA 398-10 in a solvent comprising 25 ml methanol and 25 ml acetone; evaporating the solvent from the beads; overcoating in the same apparatus the bodies with a 0.01mm (0.4 mil) thick membrane formed from a solution consisting of 1.8 g ethyl cellulose and 0.09 g lactose in 100 ml dichloromethane, and then evaporating the dichloromethane. 250 mg of the bodies, together with 1 mg of pure pyridostigmine bromide, were then placed into a 1.5 mm (cc) reservoir defined on one side by an impermeable polyethylene disc approximately 3 mm thick with a delivery port through it, and defined on the other side by a 0.013 mm (0.5 mil) thick cellulose acetate semipermeable membrane made and sold by Eastman Kodak Co. under the designation CA 383-40. The semipermeable membrane side of the device was then contacted with water, and the rate and concentration of solution delivered from the delivery port were determined. The rate of release of the drug from the device is plotted in FIG. 7. The release rate was constant for approximately eight hours while delivering the solution at a concentration of about 2 g/L. This concentration was at least 250 times less than the saturation concentration of pyridostigmine bromide, which is greater than 500 g/L.

Examples 2-4

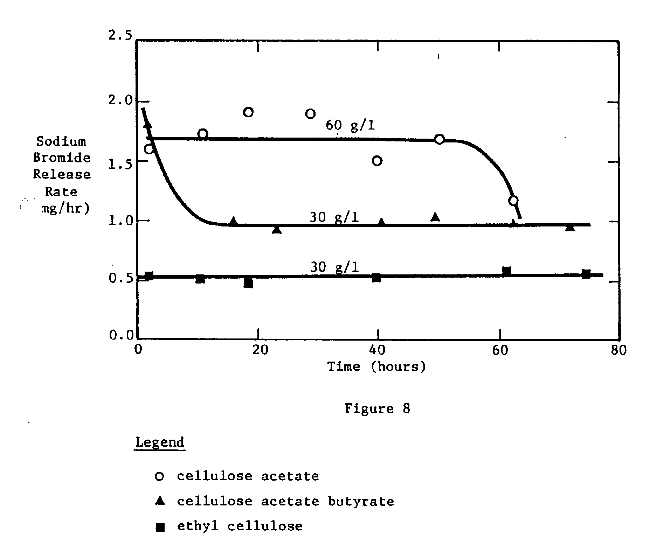

Osmotic bodies were prepared as in Example 1, except that the agent was sodium bromide and three types of osmotic bodies were prepared, each with a different semipermeable membrane: cellulose acetate, cellulose acetate butyrate, and ethyl cellulose, each containing a 19:1 mixture of polymer to lactose. These bodies were placed into identical controlled-release devices as those in Example 1 as follows: in one reservoir, 325 mg, comprising about 300 bodies, of the cellulose acetate bodies were placed; in a second reservoir, 250 mg, comprising about 160 bodies, of the cellulose acetate butyrate bodies were placed; and in a third reservoir, 250 mg, comprising about 180 bodies, of the ethyl cellulose bodies were placed. In each reservoir, 1.1 g of potassium biphthalate soluble filler was also placed. The release rates of sodium bromide from the three devices are shown in FIG. 8. Because of the different types and quantities of osmotic bodies in each device, the release rates and concentrations of sodium bromide released were different, as shown in FIG. 8. In each case, the release rate was constant, and the concentration was from 30 to 60 g/L, far below the saturation concentration of sodium bromide (700 g/L).

Example 5

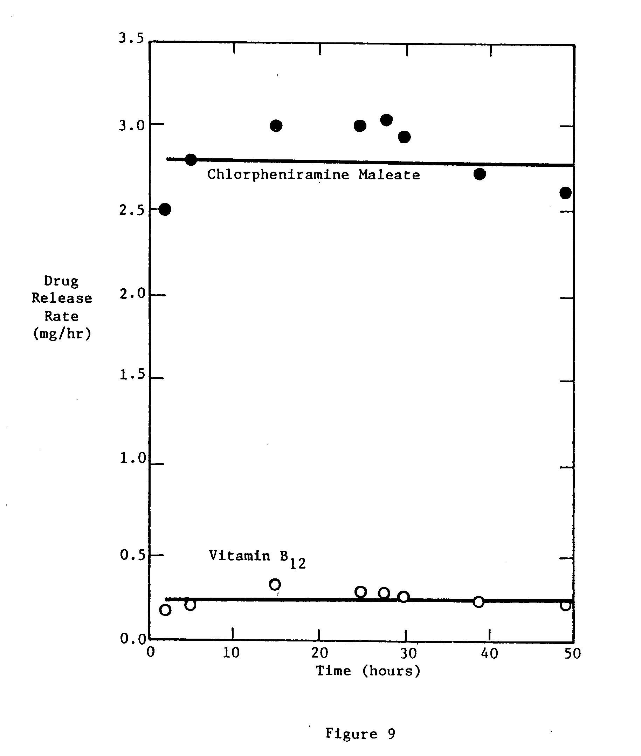

Two types of osmotic bodies were prepared as in Example 1, except that the agents were chlorpheniramine maleate (an antihistamine, hereafter "CM") and vitamin $B_{12}$ (hereafter "$B_{12}$"). 250 mg of each type of body, comprising about 270 $B_{12}$ bodies and 180 CM bodies, together with 1 g potassium biphthalate filler, were placed into a single reservoir of devices identical to those in Example 1. The release rate of each drug from this single reservoir was determined, and is shown in FIG. 9. As shown, the release rates were constant for at least 50 hours, and each drug was released at a lower concentration than saturation: 70 g/L for CM (saturation concentration 240 g/L) and 7 g/L for $B_{12}$ (Saturation concentration 12 g/L). Thus, the ratio of release rates of CM to $B_{12}$ was 10:1 instead of 20:1, the latter being the only ratio obtainable with known devices.

**Claims**

1. A device (10) for the controlled release of a less than saturated aqueous solution of at least one beneficial agent comprising a reservoir (12) defined by a surrounding wall (14;14,15) that is impermeable to said agent, at least a portion (14) of said surrounding wall comprising a membrane that is more permeable to water than to said beneficial agent, said wall further having at least one agent delivery port (16) therethrough, said reservoir containing at least one agent-containing body (20, 30), said body comprising a core (201, 301) containing a beneficial agent, characterised in that said core is surrounded by a rate-controlling membrane (202, 302) that is selected from:

a first type (a), comprising a semipermeable membrane 202 which is more permeable to water than to said beneficial agent and which is provided with at least one pre-existing agent delivery port (203) therethrough at a location predetermined before use of the device; and

a second type (b), comprising an agent-permeable membrane (302) that is either porous or non-porous.

2. The device of Claim 1 wherein said at least one agent-containing body (20, 30) comprises a mixture of said bodies having the rate-controlling membranes of types (a) and (b).

3. The device of Claim 1, additionnally comprising free beneficial agent (40) within said reservoir.

4. The device of Claim 1, additionnally comprising filler material (50) within said reservoir.

5. The device of Claim 1, additionnally comprising free beneficial agent (40) and filler material (50) within said reservoir.

6. The device of claim 1 wherein an impermeable membrane comprises a portion of said surrounding wall and said impermeable membrane has at least one agent delivery port therethrough.

7. The device of claim 1 wherein said semipermeable membrane comprises material selected from regenerated cellulose, cellulose esters, cellulose ethers, nylons, polyamides, polycarbonates, poly (dialkylsiloxanes), poly(methacrylic acid) esters, poly (acrylic acid) esters, poly(phenyleneoxides), poly (vinylalcohols), ethylene/vinyl alcohol copolymers, aromatic nitrogen-containing polymers, polymeric epoxides, polyesters, polyurethanes, and copolymers and blends thereof.

8. The device of claim 1 wherein said agent-containing body is of type (a) and comprises said beneficial agent coated with said semipermeable membrane.

9. The device of claim 1 wherein said agent-containing body is of type (a) and comprises a prill coated with one or more of said beneficial agents and overcoated with said semipermeable membrane.

10. The device of claim 1 wherein said agent-containing body is of type (b) and comprises said beneficial agent coated with said agent-permeable membrane.

11. The device of claim 1 wherein said agent-containing body is of type (b) and comprises a prill coated with said agent-permeable membrane.

**Patentansprüche**

1. Eine Einrichtung (10) für die gesteuerte Freisetzung einer geringer als gesättigten, wässrigen Lösung mindestens eines Nutzstoffes, bestehend aus einem Speicher (12), der von einer für diesen Stoff impermeablen Umgebungswand (14; 14,15) begrenzt ist von der mindestens ein Bereich (14) aus einer Membran besteht, die für Wasser mehr permeabel ist als für diesen Nutzstoff, wobei diese Wand mindestens eine durchgehende Stoffverabreichungsöffnung (16) hat, und der Speicher mindestens einen Stoff enthaltenden Körper (20, 30) aufnimmt, der einen der einen diesen Nutzstoff entaltenden Kern (201, 301) umfasst, dadurch gekennzeichnet, daß der Kern von einer geschwindigkeitssteuernden Membran (202, 302) umgeben ist, die ausgewählt ist aus:

einem ersten Typ (a), bestehend aus einer semipermeablen Membran (202) die für Wasser mehr permeabel ist als für diesen Nutzstoff und die an einer vor der Benutzung dieser Einrichtung vorgegebenen Stelle mit mindestens einer bereits vorhandenen durchgehenden, Stoffverabreichungsöffnung (203) versehen ist; und

einem zweiten Typ (b), umfassend eine für den Stoffpermeable Membran (302), die entweder porös oder nicht-porös ist.

2. Die Einrichtung nach Anspruch 1, wobei dieser mindestens eine Stoff enthaltende Körper (20, 30) eine Mischung dieser Körper mit geschwindigkeitssteuerden Membranen der Typen (a) und (b) umfasst.

3. Die Einrichtung nach Anspruch 1, welche in dem Speicher zusätzlich freien Nutzstoff (40) enthält.

4. Die Einrichtung nach Anspruch 1, welche in dem Speicher zusätzlich Füllstoff (50) enthält.

5. Die Einrichtung nach Anspruch 1, welche in dem Speicher zusätzlich freien Nutzstoff (40) und Füllstoff (50) enthält.

6. Die Einrichtung nach Anspruch 1, bei der eine impermeable Membran einen Teil dieser Umgebungswand umfasst und diese impermeable Membrane mindestens eine durchgehende Stoffverabreichungs- Öffnung aufweist.

7. Die Einrichtung nach Anspruch 1, wobei diese semipermeable Membran aus Material besteht, welches ausgewählt ist aus regenerierter Cellulose, Celluloseester, Celluloseether, Nylon, Polyamiden, Polycarbonaten, Polydiakylsiloxanen, Polymethacrylsäureestern, Polyacrylsäureestern, Polyphenyllenoxide, Polyvinylalkohole, Ethylen/Vinylalkoholcopolymere, aromatische, Stickstoff enthaltende Polymere, Polymerepoxide, Polyester, Polyurethane und Copolymere und Mischungen derselben.

8. Die Einrichtung nach Anspruch 1, wobei dieser Stoff enthaltende Körper vom Typ (a) ist und diesen Nutzstoff beschichtet mit dieser semipermeablen Membran enthält.

9. Die Einrichtung nach Anspruch 1, bei der dieser Stoff enthaltende Körper vom Typ (a) ist und ein Kügelchen umfasst, das mit einem oder mehreren Nutzstoffen beschichtet und mit dieser semipermeablen Membran überbeschichtet ist.

10. Die Einrichtung nach Anspruch 1, bei der dieser Stoff enthaltende Körper vom Typ (b) ist und diesen Nutzstoff beschichtet mit dieser stoffpermeablen Membran enthält.

11. Die Einrichtung nach Anspruch 1, bei der dieser Stoff enthaltende Körper vom Typ (b) ist und ein mit dieser stoffpermeablen Membran beschichtetes Kügelchen umfasst.

## Revendications

1. Dispositif (10) pour la libération réglée d'une solution aqueuse moins-que-saturée d'au moins un agent utile, comprenant un réservoir (12) délimité par une paroi d'entourage (14; 14, 15) qui est imperméable audit agent, au moins une partie (14) de ladite paroi d'entourage comprenant une membrane qui est plus permémable à l'eau qu'audit agent utile, ladite paroi comportant en outre au moins un orifice (16) de distribution d'agent la traversant, ledit réservoir contenant au moins un corps (20, 30) contenant un agent, ledit corps comprenant un noyau (201, 301) contenant ledit agent utile, caractérisé en ce que ledit noyau est entouré d'une membrane (202, 302) régissant la vitesse, qui est choisie parmi:

un premier type (a) comprenant une membrane semiperméable (202) qui est plus permémable à l'eau qu'audit agent utile et qui est munie d'au moins un orifice (203) de distribution d'agent préexistant, la traversant en un emplacement préétabli avant l'utilisation du dispositif; et

un second type (b) comprenant une membrane (302) permable à l'agent, qui est soit poreuse, soit non poreuse.

2. Dispositif selon la revendication 1, dans lequel ledit au moins un corps (20, 30) contenant un agent comprend un mélange desdits corps comportant les membranes de types (a) et (b) régissant la vitesse.

3. Dispositif selon la revendication 1, comprenant en outre de l'agent utile libre (40) à l'intérieur dudit réservoir.

4. Dispositif selon la revendication 1, comprenant en outre une substance de charge (50) à l'intérieur dudit réservoir.

5. Dispositif selon la revendication 1, comprenant en outre de l'agent utile libre (40) et de la substance de charge (50) à l'intérieur dudit réservoir.

6. Dispositif selon la revendication 1, dans lequel une membrane imperméable constitue une partie de ladite paroi d'entourage, et ladite membrane imperméable comporte au moins un orifice de distribution d'agent la traversant.

7. Dispositif selon la revendication 1, dans lequel ladite membrane semi-perméable comprend un matériau choisi parmi la cellulose régénérée, des esters de cellulose, éthers de cellulose, Nylons, polyamides, polycarbonates, poly(dialkylsiloxane)s, poly(ester d'acide méthacrylique)s, poly(ester d'acide acrylique)s, poly(oxyde de phénylène)s, poly(alcool vinylique)s, copolymères éthylène/alcool vinylique, polymères azotés aromatiques, époxydes polymères, polyesters, polyuréthannes et des copolymères et mélanges de ceux-ci.

8. Dispositif selon la revendication 1, dans lequel ledit corps contenant un agent est du type (a) et comprend ledit agent utile revêtu avec ladite membrane semiperméable.

9. Dispositif selon la revendication 1, dans lequel ledit corps contenant un agent est du type (a) et comprend un grain revêtu avec un ou plusieurs desdits agents utiles et recouvert avec ladite membrane semi-perméable.

10. Dispositif selon la revendication 1, dans lequel ledit corps contenant un agent est du type (b) et comprend ledit agent utile revêtu avec ladite membrane perméable à l'agent.

11. Dispositif selon la revendication 1, dans lequel ledit corps contenant un agent est du type (b) et comprend un grain revêtu avec ladite membrane permémable à l'agent.

Figure 1

Figure 2

Figure 3

Figure 4

Figure 5

Figure 6

Figure 7

Figure 8

Legend

O  cellulose acetate

▲  cellulose acetate butyrate

■  ethyl cellulose

Figure 9